# EUROPÄISCHE PATENTSCHRIFT

(11) **EP 2 598 474 B1**
(45) Veröffentlichungstag und Bekanntmachung des Hinweises auf die Patenterteilung: **18.06.2014**
(21) Anmeldenummer: 11743991.9
(22) Anmeldetag: 26.07.2011
(51) Int. Cl.: C07C 201/08

(54) **VERFAHREN ZUR KONTINUIERLICHEN HERSTELLUNG VON NITROBENZOL**
METHOD FOR CONTINUOUSLY PRODUCING NITROBENZENE
PROCÉDÉ POUR FABRIQUER EN CONTINU DU NITROBENZÈNE

(30) Priorität: 30.07.2010 DE 102010038678
(43) Veröffentlichungstag der Anmeldung: 05.06.2013
(73) Patentinhaber: Bayer Intellectual Property GmbH, 40789 Monheim (DE)
(72) Erfinder: KNAUF, Thomas, 41542 Dormagen (DE); MERKEL, Michael, 40223 Düsseldorf (DE); RAUSCH, Andreas Karl, 41564 Kaarst (DE); LEHNER, Peter, 77005 Houston, TX (US); MÜNNIG, Jürgen, 41564 Kaarst (DE)
(74) Vertreter: BIP Patents
(86) Internationale Anmeldenummer: PCT/EP2011/062833
(87) Internationale Veröffentlichungsnummer: WO 2012/013678

(56) Entgegenhaltungen:
- DE-A1-102009 005 324
- US-A- 2 431 585

## Beschreibung

Die vorliegende Erfindung betrifft ein kontinuierliches Verfahren zur Herstellung von Nitrobenzol durch Nitrierung von Benzol mit Salpetersäure oder Gemischen aus Salpetersäure und Schwefelsäure zu einem Roh-Nitrobenzol, Waschen des Roh-Nitrobenzols mittels mindestens jeweils einer sauren, alkalischen und neutralen Wäsche, wobei ein vorgereinigtes Nitrobenzol erhalten wird, welches neben Nitrobenzol mindestens noch Leichtsieder, ggf. Mittelsieder, sowie Hochsieder und Salze enthält, worin das vorgereinigte Nitrobenzol weiter gereinigt wird durch Abtrennung von Leichtsiedern in einem Destillationsapparat durch Verdampfung der Leichtsieder und Abtrennung von Nitrobenzol aus dem so erhaltenen weiter aufgereinigten Nitrobenzol in einem Destillationsapparat durch teilweise Verdampfung von Nitrobenzol, wobei Rein-Nitrobenzol dem Destillationsapparat gasförmig entnommen und anschließend kondensiert wird, und wobei der nicht verdampfte Teil des weiter aufgereinigten Nitrobenzols in eine beliebige Stelle der Wäsche zurückgeführt wird.

Nitrobenzol ist ein wichtiges Zwischenprodukt der chemischen Industrie, das insbesondere zur Herstellung von Anilin und damit zur Herstellung von Methylendiphenyldiisocyanat (MDI) und den darauf basierenden Polyurethanen benötigt wird.

Die Nitrierung von Benzol mit Salpetersäure zu einem Roh-Nitrobenzol war bereits Gegenstand zahlreicher Veröffentlichungen und Patentanmeldungen. Die heute gängigen Verfahren entsprechen im Wesentlichen dem Konzept der adiabaten Nitrierung von Benzol durch ein Gemisch von Schwefel- und Salpetersäure (sog. *Mischsäure*). Ein solches Verfahren wurde erstmals in US 2,256,999 beansprucht und ist in heutigen Ausführungsformen beispielsweise in EP 0 436 443 B1**,** EP 0 771 783 B1 und US 6 562 247 B2 beschrieben. Die Verfahren mit adiabater Reaktionsführung zeichnen sich insbesondere dadurch aus, dass keine technischen Maßnahmen ergriffen werden, um der Reaktionsmischung Wärme zu- oder abzuführen.

Auch sind isotherme Verfahren zur Nitrierung von Benzol mit Mischsäure beschrieben, wie beispielsweise in EP 0 156 199 B1 beschrieben.

Auch sind Verfahren zur Nitrierung von Benzol bekannt, die ohne den Einsatz von Schwefelsäure auskommen. Diese sind beispielsweise beschrieben in US 2 739 174 oder US 3 780 116.

Grundsätzlich sind auch Gasphasenverfahren zur Nitrierung von Benzol mit Salpetersäure oder Stickstoffoxiden möglich, jedoch sind die dadurch erzielbaren Ausbeuten noch gering (EP 0 078 247 B1**,** EP 0 552 130 B1).

All den Verfahren ist gemein, dass als Reaktionsprodukt zunächst ein Roh-Nitrobenzol entsteht, das als Verunreinigungen Salpetersäure und - sofern mit Mischsäure nitriert wurde - Schwefelsäure enthält, sowie als organische Verunreinigungen Dinitrobenzol sowie nitrierte Oxidationsprodukte des Benzols, insbesondere nitrierte Phenole (Nitrophenole). Auch enthält es organische Verbindungen, die aus den Verbindungen entstanden, die als Verunreinigungen im eingesetzten Benzol enthalten waren (WO 2008/148608 A1). Darüber hinaus enthält das Roh-Nitrobenzol auch Metallsalze, die in den Säureresten oder im Roh-Nitrobenzol gelöst vorliegen können (DE 10 2007 059 513 A1).

Zahlreiche Untersuchungen zielten in der Vergangenheit darauf ab, die Qualität des Roh-Nitrobenzols zu verbessern und somit die Ausbeute bezüglich Benzol und Salpetersäure zu erhöhen. Dank dieser Entwicklungen sind die heutigen adiabaten Flüssigphasenverfahren so ausgereift, dass es allen gelingt, ein Roh-Nitrobenzol herzustellen, das einen geringen Gehalt an Nebenprodukten aufweist, also nur zwischen 100 ppm und 300 ppm an Dinitrobenzol und zwischen 1500 ppm und 2500 ppm an Nitrophenolen enthält, wobei Pikrinsäure einen Anteil von 10 % bis 50 % an den Nitrophenolen annehmen kann.

Das Roh-Nitrobenzol weist als Verunreinigungen noch Wasser, Benzol sowie Nitrophenole und Dinitrobenzol und - sofern mit Mischsäure nitriert wurde - Schwefelsäure auf, die durch geeignete Aufarbeitungsverfahren wie z. B. Wasch- und Destillationsstufen abgetrennt werden. Eine mögliche Ausführungsform dieser Aufarbeitung ist in EP 1 816 117 B1 beschrieben, wo das Nitrobenzol einer sauren Wäsche, einer alkalischen Wäsche, einer neutralen Wäsche und abschließend einer destillativen Reinigung unterzogen wird. Dabei besteht die Aufgabe der in EP 1 816 117 B1 beschriebenen Destillation lediglich darin, Wasser und Benzol aus dem Nitrobenzol zu entfernen und nicht darin, Nitrobenzol möglichst vollständig zu verdampfen. Das "Rein"-Nitrobenzol eines solchen Verfahrens ist daher das *Sumpfprodukt* der Destillation und ist gemäß EP 1 816 117 B1 dadurch charakterisiert, dass es eine Leitfähigkeit von < 50 µS/cm, bevorzugt von < 25 µS/cm und besonders bevorzugt von < 10 µS/cm aufweist (Methode zur Leitfähigkeitsmessung siehe ebendort, Absatz [0025]). Die Leitfähigkeitsmessung als alleiniges Maß für die Nitrobenzolreinheit zu wählen ist nachteilig, da diese nur wasserlösliche und dissoziierbare Verbindungen ausweist. Hochmolekulare Verbindungen, wie z. B. nitrierte Biphenyle (siehe Beispiel 1) sowie nicht dissoziierbare MetallVerbindungen wie Eisenoxide, sowie schlecht wasserlösliche Verbindungen wie Calciumsulfat, Silikate oder Metallkomplexe werden nicht erfasst und könnten somit unbemerkt im Nitrobenzol verbleiben, da das "Rein"-Nitrobenzol in EP 1 816 117 B1 nur das Sumpfprodukt der Destillation darstellt. Hochsiedende organische Verbindungen und Salze können mit einer solchen Destillation nicht abgetrennt werden. Insbesondere Salze können bei der weiteren Verwendung des gereinigten Nitrobenzols, etwa in der Hydrierung zu Anilin (siehe unten), große Probleme bereiten, indem diese zur Bildung von Ablagerungen in Apparaten (z. B. Verdampfern) führen oder - wenn sie bis in die Reaktionszone gelangen - eine vorzeitige Desaktivierung des Katalysators zur Folge haben können.

Wenn in der Literatur zur Nitrobenzolherstellung von einer Destillation des Nitrobenzols gesprochen wird, bezieht sich dies fast immer auf die destillative Abtrennung von Wasser und Benzol, während das Nitrobenzol selbst in den im Stand der Technik üblichen Verfahren das *Sumpfprodukt* einer solchen Destillation ist. Vor einer Verdampfung des Nitrobenzols selbst wird in manchen Schriften sogar aus Sicherheitsgründen ausdrücklich abgeraten. So beschreibt US 4,021,498 ein spezielles Verfahren zur adiabaten Nitrierung von Benzol, dass darauf abzielt, den Gehalt an dinitrierten Verbindungen im Produkt zu minimieren (siehe Spalte 2, Z. 18 bis 29). US 4,021,498 weist auf die Gefahren, die mit einer Verdampfung des Nitrobenzols selbst einhergehen, eindrücklich hin (Spalte 2, Z. 7 bis 17) und lehrt damit von einer Reinigung des Nitrobenzols durch Verdampfung und Rekondensation weg.

Nur wenige Druckschriften über die Herstellung bzw. Reinigung von Nitrobenzol lehren tatsächlich eine Verdampfung des Nitrobenzols selbst. So beschreibt US 1,793,304 ein Verfahren zur Reinigung von (u. a.) Nitrobenzol, bei dem verunreinigtes Nitrobenzol zunächst durch Filtration durch sog. Bleicherdefilter ("fuller's earth") und anschließend durch eine Wärmebehandlung mit Aluminiumoxid oder anderen basischen Oxiden bei 80 °C bis 100 °C gereinigt wird (S. 1, Z. 63 bis 71). An die Behandlung mit Aluminiumoxid schließt sich bevorzugt eine Destillation im partiellem Vakuum bei Sumpftemperaturen von 140 °C bis 160 °C an (S. 1, Z. 74 bis 79). Weitere Details zur Ausführung der Destillation nennt die Druckschrift nicht; jedoch kann aufgrund des Siedepunktes von Nitrobenzol bei Normaldruck (211 °C) davon ausgegangen werden, dass Nitrobenzol im genannten Temperaturbereich und im "partiellen Vakuum" tatsächlich verdampft wird. Angaben zur Wiedergewinnung der bei der Destillation eingesetzten Energie liefert diese Schrift nicht.

US 2,431,585 beschreibt eine Gasphasennitrierung von Benzol mit abschließender Destillation des Nitrobenzols über Kopf der Destillationskolonne 14 (siehe Figur). Eine partielle Ausschleusung des Destillationssumpfes ist nicht vorgesehen.

CH 186266 offenbart ein Verfahren zur Reinigung von technischem Nitrobenzol, bei dem Verunreinigungen wie Dinitrobenzol vor dem Abdestillieren des Nitrobenzols durch chemische Reaktion mit basischen Verbindungen und organischen Stoffen (siehe Unteranspruch 1, Beispiele 2 bis 6) in andere, leicht abtrennbare Verbindungen überführt werden. Als Destillationsbedingungen werden 122 °C und 66 mbar angegeben (siehe Beispiel 1). Auf dem Niveau dieser Destillationstemperaturen lässt sich die Kondensationswärme nicht sinnvoll, z. B. in Form von nutzbarem Dampf, wiedergewinnen.

RU 2 167 145 C1 beschreibt die Abtrennung von hoch siedenden Verbindungen - insbesondere schwefelhaltigen organischen Verbindungen - von Nitrobenzol durch Rektifikation des Nitrobenzols, wobei Nitrobenzol über Kopf der Rektifizierkolonne entnommen wird. In dieser Schrift wird jedoch nicht auf die besondere Problematik von Salzen im Roh-Nitrobenzol eingegangen, die zur Verstopfung von Packungen und Wärmeaustauschern führen können. Auch sind die in RU 2167145 C 1 gewählten Destillationsbedingungen nachteilig, da nur niedrige Drücke (20 mm Hg bis 80 mm Hg, entsprechend 27 mbar bis 107 mbar; siehe Anspruch 1) angewandt werden können, was zu Kondensationstemperaturen von lediglich 99 °C bis 134 °C führt, wie der Fachmann durch Berechnung (beispielsweise durch Berechnung der Sättigungsdampfdruckkurve unter Zuhilfenahme der Antoine-Gleichung, siehe hierzu I. Brown, Australian Journal of Scientific Research, Series B: Biological Sciences (1952), 5A, 530 - 540) leicht feststellen kann. Auf diesem Niveau dieser Kondensationstemperaturen lässt sich die Kondensationswärme nicht sinnvoll, z. B. in Form von nutzbarem Dampf, wiedergewinnen.

CN 100999472 A beschreibt die Aufarbeitung des Sumpfrückstandes der Nitrobenzol-Destillation zur Gewinnung von m-Dinitrobenzol, ohne genauer auf die Bedingungen der Destillation des Nitrobenzols einzugehen.

Das gereinigte Nitrobenzol (Rein-Nitrobenzol) wird überwiegend zur Herstellung von Anilin eingesetzt, die heute wiederum überwiegend durch katalytische Hydrierung des Nitrobenzols in der Gasphase mit Wasserstoff erfolgt. Zur Überführung in die Gasphase kann Nitrobenzol entweder verdampft (EP 0 696 574 B1, Absatz [0024]) oder in einen heißen Gasstrom hinein, bevorzugt in einen Wasserstoffstrom hinein verdüst (DE-OS-1 809 711, DE 10 2006 035 203 A1, Absatz [0053]) werden. Eine Verdampfung in den Frischwasserstoffstrom hinein gilt als vorteilhaft, da sich dadurch deutlich geringere Ablagerungen im Reaktor und in den Zuleitungen bilden sollen (EP 0 696 574 B1, Absatz [0024]). Jedoch vermeidet eine Verdampfung in den Frischwasserstoffstrom hinein die Ausbildung von Ablagerungen im Reaktor nicht vollständig, da im Nitrobenzol befindliche Metallverbindungen, Salze und Hochsieder weiterhin dort anfallen werden. Der Anfall von Feststoffen (wie hoch siedenden organischen Verbindungen und Salzen) im Reaktor ist nachteilig, da sich dadurch die Reinigungsintervalle des Reaktors verkürzen und Katalysator vorzeitig desaktiviert werden kann. Wird das Nitrobenzol in einem herkömmlichen Verdampfer verdampft, kann man zwar Probleme mit Ablagerungen im Reaktor minimieren, verlagert diese jedoch auf die Verdampferstufe: Häufige erforderliche Reinigungen des Verdampfers führen immer wieder zu Unterbrechungen des kontinuierlichen Betriebs der Anilinanlage. Hinzu kommen unter Umständen, abhängig von der Qualität des eingesetzten Nitrobenzols, die oben genannten Sicherheitsprobleme. Es besteht daher Bedarf an einer Herstellmethode für Nitrobenzol, die dieses von vorneherein mit der nötigen Reinheit zur Verfügung stellt, ohne dass in Verfahrensschritten der Anilinherstellung noch weitere Reinigungsschritte erforderlich werden.

Die Hydrierung von Nitrobenzol zu Anilin kann an ortsfesten Katalysatorbetten in Rohrbündelreaktoren (DE-AS-2 201 528) oder Hordenreaktoren (EP 0 696 574 B1, Absatz [0021]) durchgeführt werden oder auch in Wirbelbettreaktoren (DE-AS-1 114 820). In zahlreichen Publikationen sind Katalysatoren für die Gasphasenhydrierung von Nitroaromaten beschrieben. Die hydrieraktiven Elemente umfassen Pd, Pt, Ru, Fe, Co, Ni, Cu, Mn, Re, Cr, Mo, V, Pb, Ti, Sn. Dy. Zn, Cd, Ba, Cu, Ag, Au. Ebenso können diese Elemente in Form ihrer Verbindungen, zum Beispiel als Oxide, Sulfide oder Selenide und auch in Form einer Raney-Legierung sowie auf Trägern, wie Al₂O₃, Fe₂O₃/Al₂O₃, SiO₂, Silikaten, Kohlen, TiO₂, Cr₂O₃ als Hydrierkatalysator eingesetzt werden.

Die Reaktoren können unter Anwendung von Kühlung isotherm (DE-AS-2 201 528) oder auch adiabat (EP 0 696 574 B1) betrieben werden. Auch Kombinationen von isothermen und adiabaten Reaktionsabschnitten sind möglich (GB 1 452 466).

Allen Druckschriften, in denen die Verdampfung des Nitrobenzols offenbart wird, sei es im Rahmen einer Nitrobenzolsynthese in einer Destillationsapparatur, oder im Rahmen der Verdampfung des Edukts Nitrobenzol in einer Gasphasen-Anilinsynthese, ist gemein, dass die *möglichst vollständige* Verdampfung des Nitrobenzols angestrebt wird. Dies hat einerseits zur Folge, dass Sicherheitsprobleme bei der Eindampfung auftreten können (siehe oben) und andererseits, dass es zu Ablagerungen an hoch siedenden organischen Nebenkomponenten und Salzen in Verdampfern und Destillationsapparaturen kommt, die den Betrieb empfindlich stören und zu Produktionsunterbrechungen führen können.

Eine Aufgabe der vorliegenden Erfmdung war es daher, die Aufarbeitung von Roh-Nitrobenzol so zu gestalten, dass diese möglichst störungsfrei (kein Anlagenausfall etwa durch notwendig gewordene Entfernung von Salzablagerungen in einer Destillationskolonne), sicher (keine Probleme durch Anreicherung potenziell explosionsgefährlicher Verbindungen im Sumpf einer Destillationskolonne) und energieeffizient (möglichst optimale Nutzbarmachung von im Prozess anfallender Wärme) durchgeführt werden kann.

Ferner war es Aufgabe der vorliegenden Erfindung, ein Rein-Nitrobenzol bereitzustellen, in welchem hoch siedende organische Verbindungen und Salze entweder vollständig entfernt oder zumindest soweit abgereichert sind, dass dieses Rein-Nitrobenzol ohne Nachteile in weiteren Verwendungen eingesetzt werden kann.

Insbesondere war es auch Aufgabe der vorliegenden Erfindung, ein Rein-Nitrobenzol bereitzustellen, mit dem ein Anilinverfahren mit hohen Reaktor-Standzeiten (= lange Reinigungsintervalle) betrieben werden kann. Zur Bereitstellung eines solchen Nitrobenzols kann die Qualität des Nitrobenzols nicht - wie im Stand der Technik beschrieben - allein aufgrund seiner Leitfähigkeit beurteilt werden. So wurde gefunden, dass ein Nitrobenzol, dessen Leitfähigkeit weniger als 10 µS/cm betrug und demnach im Sinne der EP 1 816 117 B1 als rein zu bezeichnen wäre, noch bis zu 200 ppb (0,2 ppm) an Natrium enthalten kann. Der Einsatz eines solchen Nitrobenzols in einem Gasphasen-Anilinverfahren ist nachteilig, da es zu einer Verunreinigung der Nitrobenzol-Erhitzer und -Verdampfer kommen kann, bzw. im Falle einer Verdüsung des Nitrobenzols zu einer Verunreinigung der Katalysatoroberfläche, die zu einer Verringerung der Selektivität und/oder der Lebensdauer des Katalysators führen kann (siehe Beispiele 4 und 5).

Es wurde gefunden, dass die Aufgabenstellung gelöst werden kann durch ein Verfahren zur Herstellung von Nitrobenzol durch
a) Nitrierung von Benzol mit Salpetersäure oder Gemischen aus Salpetersäure und Schwefelsäure und anschließende Abtrennung überschüssiger in der Nitrierung eingesetzter Säure, wobei Roh-Nitrobenzol erhalten wird,
b) Waschen des Roh-Nitrobenzols aus Schritt a) mittels mindestens jeweils einer sauren, alkalischen und neutralen Wäsche in bevorzugt dieser Reihenfolge, wobei nach Abtrennung der in der letzten Wäsche eingesetzten Waschflüssigkeit ein vorgereinigtes Nitrobenzol erhalten wird, welches neben Nitrobenzol mindestens noch Leichtsieder, ggf. Mittelsieder, sowie Hochsieder und Salze enthält,
c) Abtrennung von Leichtsiedern aus dem vorgereinigten Nitrobenzol aus Schritt b) in einem Destillationsapparat durch Verdampfung der Leichtsieder, wobei das Sumpfprodukt Nitrobenzol an Leichtsiedern abgereichert und auf diese Weise weiter aufgereinigt wird,
d) teilweise Abtrennung von Nitrobenzol aus dem weiter aufgereinigten Nitrobenzol aus Schritt c) in einem Destillationsapparat durch teilweise Verdampfung von Nitrobenzol, und zwar bevorzugt durch Verdampfung von > 80 Massen-% bis ≤ 99,9 Massen-%, bevorzugt zwischen > 90 Massen-% und ≤ 95 Massen-%, des weiter aufgereinigten Nitrobenzols, bezogen auf die Gesamtmasse des weiter aufgereinigten Nitrobenzols, wobei Rein-Nitrobenzol dem Destillationsapparat gasförmig entnommen und anschließend kondensiert wird und unverdampftes Nitrobenzol, ggf. Mittelsieder, sowie Hochsieder und Salze aus dem Sumpf des Destillationsapparates teilweise bis vollständig, bevorzugt vollständig, ausgeschleust werden, und
e) Rückführung des nicht verdampften Teils des weiter aufgereinigten Nitrobenzols aus Schritt d) in eine beliebige Stelle von Schritt b), bevorzugt in die saure Wäsche.

*Überschüssige in der Nitrierung eingesetzte Säure* bezeichnet dabei jede Art nicht umgesetzter Säure, also
ggf. Salpetersäure bei Durchführung der Nitrierung mit lediglich Salpetersäure
oder
Schwefelsäure oder ggf. Salpetersäure und Schwefelsäure bei Durchführung der Nitrierung mit Gemischen aus Salpetersäure und Schwefelsäure.

Als *Leichtsieder* werden im Rahmen dieser Erfindung alle Verbindungen und azeotrop siedende Gemische von Verbindungen bezeichnet, deren Siedepunkte unterhalb dem von Nitrobenzol unter den gewählten Destillationsbedingungen liegen. Der Hauptbestandteil der Leichtsieder ist nicht vollständig umgesetztes Benzol. Weitere typische Leichtsieder sind n-Hexan, Cyclohexan, n-Heptan, Methylcyclohexan, Bicycloheptan und die isomeren Dimethylpentane.

Als *Mittelsieder* werden im Rahmen dieser Erfindung alle Verbindungen und azeotrop siedende Gemische von Verbindungen bezeichnet, deren Siedepunkte oberhalb dem von Nitrobenzol unter den gewählten Destillationsbedingungen, aber unterhalb von 350 °C bei Normaldruck liegen. Typische Mittelsieder sind die isomeren Dinitrobenzole.

Als *Hochsieder* werden im Rahmen dieser Erfindung alle Verbindungen und azeotrop siedende Gemische von Verbindungen bezeichnet, deren Siedepunkte bei Normaldruck oberhalb von 350 °C liegen. Die Siedepunkte solcher Verbindungen sind so hoch, dass bei einer katalysierten Gasphasen-Hydrierung zu Anilin, welches die bevorzugte Verwendung erfindungsgemäß hergestellten Nitrobenzols ist, eine Verunreinigung und Desaktivierung des Katalysators nicht ausgeschlossen werden kann. Typische Hochsieder weisen mehr als einen aromatischen Ring auf und umfassen beispielsweise nitrierte Biphenyle, sowie nitrierte Hydroxy-Biphenyle.

Typische *Salze,* die mit dem erfindungsgemäßen Verfahren aus dem Nitrobenzol abgetrennt werden, sind die Natrium- und Calciumsalze von Nitrit, Nitrat, Sulfat und Oxalat sowie Siliziumoxid und Eisenoxid.

Durch die erfindungsgemäße Vorgehensweise werden außerordentlich hohe Reinheitsgrade des Nitrobenzols erzielt. Nach dem erfindungsgemäßen Verfahren hergestelltes Nitrobenzol hat einen Reinheitsgrad in Bezug auf organische Verunreinigungen (im Wesentlichen die oben genannten Mittelsieder) von > 99,9500 % (entsprechend < 500 ppm Verunreinigungen), bevorzugt von > 99,9900 % (entsprechend < 100 ppm Verunreinigungen), besonders bevorzugt von größer > 99,9990 % (entsprechend < 10 ppm Verunreinigungen) (bevorzugt bestimmt mittels Gaschromatographie), und enthält maximal 0,1 ppm, bevorzugt maximal 0,05 ppm, besonders bevorzugt maximal 0,01 ppm anorganische Verunreinigungen (Salze, bestimmt als Kationen, per Atomabsorptionspektrometrie (Inductive Coupled Plasma, ICP). (Sofern nicht anders vermerkt, beziehen sich Gehaltsangaben in % und ppm immer auf die Masse).

Ein nach dem erfindungsgemäßen Verfahren hergestelltes, hoch reines Nitrobenzol eignet sich besonders für die Verwendung in der Hydrierung zu Anilin.

Schritt a) des erfindungsgemäßen Verfahrens kann im Prinzip nach allen im Stand der Technik bekannten Methoden erfolgen. Bevorzugt ist die Umsetzung von Benzol mit einem Gemisch aus Salpetersäure und Schwefelsäure unter adiabater Verfahrensführung, wie sie in DE 10 2008 048713 A1, Absatz [0024] beschrieben ist.

Schritt b) des erfindungsgemäßen Verfahrens ist im Stand der Technik grundsätzlich bekannt. Bei dieser dreistufigen Wäsche wird das in Schritt a) erhaltene Roh-Nitrobenzol mit geeigneten Waschflüssigkeiten (d. h. Waschwässer eines der jeweiligen Stufe entsprechenden pH-Werts) in drei Waschschritten gewaschen, wobei die einzelnen Waschschritte prinzipiell in beliebiger Reihenfolge durchgeführt werden können. Bevorzugt ist jedoch die Reihenfolge (1) saure Wäsche - (2) alkalische Wäsche - (3) neutrale Wäsche. Besonders bevorzugt erfolgt Schritt b) nach der in den Absätzen [0008] bis [0012] von EP 1 816 117 B1 beschriebenen Vorgehensweise. Besonders bevorzugt wird in der neutralen Wäsche in Schritt b) eine Elektrophorese eingesetzt, wie sie in EP 1 816 117 B1, Absatz [0013] beschrieben ist.

In Schritt c) des erfindungsgemäßen Verfahrens wird als Destillationsapparat bevorzugt eine Rektifizierkolonne eingesetzt, d. h. ein Apparat, in dem mindestens eine theoretische Trennstufe realisiert wird und bei dem ein flüssiger Rücklauf oben in die Kolonne eingespeist wird. Das Rücklaufverhältnis, d. h. das Verhältnis von Rücklauf zu abgezogenem Kondensat, liegt dabei bevorzugt zwischen 0,01 und 0,5, und als Rücklauf wird bevorzugt ein (oder mehrere) Leichtsieder, besonders bevorzugt Benzol, eingesetzt. Bei dem als Rücklauf bevorzugt verwendeten Benzol kann es sich um frisches Benzol handeln, oder besonders bevorzugt um Benzol, das aus dem Kopfprodukt der Rektifizierkolonne nach Abtrennung des Wassers (z. B. durch statische Phasentrennung) erhalten wurde. Bei dem als Rücklauf bevorzugt eingesetzten Benzol muss ferner nicht notwendigerweise reines Benzol eingesetzt werden. Vielmehr kann es sich auch um ein Stoffgemisch handeln, das neben Benzol zwischen 0,1 Massen-% und 10 Massen-% Nitrobenzol und/oder zwischen 0,1 Massen-% und 50 Massen-% an aliphatischen Kohlenwasserstoffen enthält, jeweils bezogen auf die Gesamtmasse des Rücklaufs. In Schritt c) des erfindungsgemäßen Verfahrens ist Nitrobenzol *das Sumpfprodukt,* d. h. nur Nitrobenzol aus dem Sumpf von Schritt c) wird in Schritt d) abdestilliert. (Eventuell können in Schritt c) vernachlässigbare Mengen an Nitrobenzol mit den Leichtsiedern mitgerissen, und, sofern diese nicht mit dem Rücklauf wieder in den Sumpf gelangen, aus dem System ausgetragen werden.)In Schritt d) der vorliegenden Erfindung werden Hochsieder und Salze abgetrennt, indem das in Schritt c) erhaltene weiter aufgereinigte Nitrobenzol einem Destillationsprozess unterworfen wird, in welchem Rein-Nitrobenzol dem Destillationsapparat gasförmig entnommen und anschließend kondensiert wird, d. h. in diesem Schritt wird das Nitrobenzol selbst abdestilliert. Bevorzugt wird das Rein-Nitrobenzol der Destillationskolonne über Kopf entnommen. Eine Seitenstromentnahme ist jedoch ebenfalls vorstellbar. Das dabei im Destillationsapparat anfallende Sumpfprodukt macht zwischen > 0,1 Massen-% und ≤ 20 Massen-%, bevorzugt zwischen > 5 und ≤ 10 Massen-%, des weiter aufgereinigten Nitrobenzols aus, das in den Destillationsapparat in Schritt d) eingeführt wird. Die erfindungsgemäße *teilweise Abtrennung von Nitrobenzol aus dem weiter aufgereinigten Nitrobenzol in einem Destillationsapparat durch teilweise Verdampfung von Nitrobenzol* bedeutet also, dass in Schritt d) zwischen > 80 Massen-% und ≤ 99,9 Massen-%, bevorzugt zwischen > 90 Massen-% und ≤ 95 Massen-%, des weiter aufgereinigten Nitrobenzols, bezogen auf die Gesamtmasse des weiter aufgereinigten Nitrobenzols, verdampft werden.

In Schritt e) wird der in Schritt d) anfallende Destillationssumpf (also der nicht verdampfte Teil des weiter aufgereinigten Nitrobenzols) einer beliebigen Stelle der Wäsche (Schritt b) zugeführt. Diese erfindungsgemäße Vorgehensweise, bei welcher der Sumpf der Destillationskolonne in Schritt d) nicht vollständig eingedampft wird, erhöht zum einen die Sicherheit des Verfahrens. Zum anderen werden Probleme mit Feststoffabscheidung in Apparaten vermieden, weil ein noch flüssiges Sumpfprodukt ausgeschleust und in die Wäsche (Schritt b)) zurückgeführt wird. Dort werden Salze ausgewaschen, und mit dem Sumpf ausgetragenes Nitrobenzol wird bis auf vernachlässigbare Restgehalte in die organische Phase der jeweiligen Waschstufe überführt, d. h. es wird in das vorgereinigte Nitrobenzol zurückgeführt. Hierdurch werden Ausbeuteverluste minimiert.

Bevorzugt wird der Druck im Destillationsschritt d) so gewählt, dass sich eine solche Kondensationstemperatur des Rein-Nitrobenzols ergibt, bei der die Kondensationswärme auf wirtschaftliche Art und Weise zur Dampfgewinnung verwendet werden kann. Die Erfindung betrifft demnach insbesondere auch ein Verfahren, bei dem in Schritt d) absolute Drücke im Bereich zwischen 150 mbar und 1000 mbar, bevorzugt zwischen 200 mbar und 600 mbar, besonders bevorzugt zwischen 400 mbar und 500 mbar, eingehalten werden und bei dem die in der Kondensation des Rein-Nitrobenzols in Schritt d) abzuführende Wärme zur Gewinnung von Dampf eingesetzt wird. Dieser in Schritt d) herrschende Druck wird dabei bevorzugt am Kondensator gemessen, in welchem das den Destillationsapparat gasförmig verlassende Rein-Nitrobenzol kondensiert wird. Alle nach dem Stand der Technik gängigen Kondensatoren können hierzu eingesetzt werden, bevorzugt sind Rohrbündel-Wärmetauscher oder Plattenwärmetauscher.

Bei solchen Drücken in Schritt d) stellen sich Kondensationstemperaturen des Rein-Nitrobenzols zwischen 140 °C und 210 °C, bevorzugt zwischen 150 °C und 190 °C, besonders bevorzugt zwischen 175 °C und 185 °C, ein. Bevorzugt wird im erfindungsgemäßen Verfahren in der Kondensation des Rein-Nitrobenzols in Schritt d), besonders bevorzugt im gesamten Schritt d), ganz besonders bevorzugt in den gesamten Schritten a) bis d), zu keinem Zeitpunkt eine Temperatur von 210 °C überschritten. Nur wenn das Rein-Nitrobenzol bei Temperaturen von maximal 210 °C kondensiert wird, kann es frei von oder mindestens sehr arm an, insbesondere organischen, hoch siedenden Verbindungen ("Hochsieder") gewonnen werden. Es wurde nämlich gefunden, dass sich bei Temperaturen > 210 °C aus Nitrobenzol Hochsieder bilden (siehe Beispiel 2). Daher gelingt die Bereitstellung von salzfreiem Nitrobenzol, das gleichzeitig auch frei von oder mindestens sehr arm an Hochsiedern ist, unter gleichzeitiger Maximierung der Ausbeute (bezogen auf Benzol) überraschenderweise nur dann, wenn die maximale Temperatur, bei der das Nitrobenzol kondensiert wird (d. i. im Allgemeinen die Flüssigphasentemperatur im Kondensator), 210 °C nicht übersteigt. Nun ist eine hohe Kondensationstemperatur aber grundsätzlich anzustreben, da nur bei einer hinreichend hohen Kondensationstemperatur die Kondensationswärme auf wirtschaftliche Weise zur Dampfgewinnung genutzt werden kann. Daher ist die tatsächlich einzustellende Kondensationstemperatur i. A. ein Kompromiss und gewährleistet bevorzugt einen absoluten Druck des gewonnenen Dampfes von mindestens 4 bar, was einer Kondensationstemperatur von 150 °C entspricht.

Da die bevorzugte Verwendung des erfindungsgemäßen Rein-Nitrobenzols die Hydrierung zu Anilin ist, muss bei der Abtrennung von Hochsiedern in Schritt d) das Augenmerk besonders auf solche Verbindungen gelegt werden, die unter den Bedingungen eines Gasphasen-Anilinverfahrens nicht gasförmig sind. Bevorzugt erfolgt die Anilinherstellung gemäß dem Verfahren aus DE 10 2006 035 203 A1. Insbesondere bevorzugt werden die dort in Absatz [0018] genannten Bereiche von Temperatur, Druck, Wassergehalt im Eduktgasstrom und Wasserstoffüberschuss eingehalten. Nitrobenzol wird bevorzugt mittels Verdüsung in die Gasphase überführt wie in Abschnitt [0053] beschrieben. Hoch siedende organische Verbindungen, die unter den Bedingungen eines solchen Gasphasen-Anilinverfahrens nicht gasförmig sind, und im vorgereinigten Nitrobenzol vorhandene Salze lassen sich in Schritt d), der der Anilinherstellung vorgelagerten Nitrobenzolaufarbeitung bevorzugt in einem Destillationsapparat ohne trennwirksame Einbauten, abtrennen. Bei einer Destillation ohne trennwirksame Einbauten spricht man auch von *Evaporation.* Bevorzugt verfügt der Destillationsapparat jedoch über Demister oder Tropfenabscheider.

Besonders bevorzugt ist demnach ein Verfahren, bei dem der Destillationsapparat in Schritt c) eine Rektifizierkolonne ist, welche unter teilweisem Rücklauf von Leichtsiedern, bevorzugt Benzol, besonders bevorzugt von Benzol enthaltend Nitrobenzol und/oder aliphatische Kohlenwasserstoffe, betrieben wird, und in Schritt d) ein Apparat ohne trennwirksame Einbauten ist. In Schritt d) kann auf einen Rücklauf (der in diesem Fall ein Teil des kondensierten Rein-Nitrobenzols wäre) auch verzichtet werden. In dieser Ausführungsform betrifft die Erfindung demnach ein Verfahren, bei dem der Destillationsapparat in Schritt c) eine Rektifizierkolonne, welche unter teilweisem Rücklauf von Leichtsiedern, bevorzugt Benzol, besonders bevorzugt von Benzol enthaltend Nitrobenzol und/oder aliphatische Kohlenwasserstoffe, betrieben wird, und in Schritt d) ein Apparat ohne trennwirksame Einbauten, welcher ohne Rücklauf kondensierten Rein-Nitrobenzols betrieben wird, ist. Durch diese besonders bevorzugte Ausgestaltung der erfindungsgemäßen zweistufigen (Schritte c) und d)) Destillation wird zum einen sichergestellt, dass die Leichtsieder effizient vom Nitrobenzol abgetrennt werden (Rektifikation in Schritt c)) und zum anderen, dass die Abtrennung von Hochsiedern und insbesondere Salzen in Schritt d) ohne die Gefahr einer Verstopfung von Packungen in der Destillationskolonne erfolgt. Ggf. in Schritt d) nicht vollständig abgetrennte Mittelsieder sind im Rein-Nitrobenzol nur in unkritischen Gehalten von < 500 ppm, bevorzugt < 100 ppm, besonders bevorzugt < 10 ppm, bezogen auf die Masse des Rein-Nitrobenzols, enthalten.

Da die Bildung von hoch siedenden organischen Verbindungen (wie die genannten nitrierten Biphenyle) auch bei niedrigeren Temperaturen als 210 °C stattfinden kann, wenn das Nitrobenzol diesen Temperaturen *zu lange* ausgesetzt ist, ist es vorteilhaft, wenn die verwendeten Apparate eine kurze Verweilzeit aufweisen. So können etwa Selbstumlaufverdampfer, z. B. sog. *Robert-Verdampfer,* als Verdampfer für die Destillationskolonnen in den Schritten c) und d) eingesetzt werden (siehe Reinhard Billet: Verdampfung und ihre technischen Anwendungen, Weinheim, 1981, S. 119 f.) In solchen Verdampfern beträgt die Verweilzeit des Nitrobenzols bevorzugt 0,1 Minuten bis 120 Minuten, besonders bevorzugt 0,1 Minuten bis 20 Minuten.

Die Abtrennung von niedrig und hoch siedenden Verbindungen und Salzen aus dem Nitrobenzol kann in einer Trennwandkolonne kombiniert werden. (Das generelle Funktionsprinzip von Trennwandkolonnen wird beispielsweise in G. Kaibel, "Distillation Columns with Vertical Partitions", Chem. Eng. Technol. 1987, 10, 92 - 98 und G. Kaibel, "Industrieller Einsatz von Trennwandkolonnen und thermisch gekoppelten Destillationskolonnen", Chemie Ingenieur Technik 2003, 75, 1165 - 1166 beschrieben.) In dieser Ausführungsform betrifft die Erfindung demnach insbesondere ein Verfahren, bei dem in den Schritten c) und d) derselbe Destillationsapparat eingesetzt wird und dieser eine Trennwandkolonne ist.

### Beispiele

### Beispiel 1 (erfindungsgemäß) : Abtrennung von Hochsiedern durch Destillation des Nitrobenzols - Schritt d)

277,6 g eines Nitrobenzols, das keine nachweisbaren Mengen an Leichtsiedern und 55 Flächen-ppm an Hochsiedern (bestimmt durch Gaschromatographie, GC) aufweist, wurden in einer Destillationsapparatur bestehend aus Glaskolben und Destillationsbrücke bei 180 °C und 400 mbar (absolut) destilliert (Schritt d) des erfindungsgemäßen Verfahrens), sodass 271,4 g an Destillat und 6,2 g als Sumpfprodukt erhalten wurden. Im Destillationssumpf wurden mittels GC ca. 3700 Flächen-ppm an Hochsiedern gefunden. In der Gruppe der Hochsieder konnten mittels GC-MS (Massenspektrometrie) 2-Nitrobiphenyl und 2,2-Dinitrobiphenyl identifiziert werden. Im Destillat (= Rein-Nitrobenzol) konnten keine Hochsieder gefunden werden, was zeigt, dass mit dem erfindungsgemäßen Verfahren ein hochsiederfreies Nitrobenzol bei niedrigen Destillationstemperaturen (< 210 °C) erhalten werden kann.

### Beispiel 2 (Vergleichsbeispiel): Entstehung von Hochsiedern durch Tempern

In einem Edelstahlautoklaven wurde Nitrobenzol einer Reinheit (GC) > 99,9900 % und mit einem Salzgehalt von < 0,1 ppm (das also im Sinne der vorliegenden Anmeldung als frei von Leichtsiedern, Hochsiedern und Salzen bezeichnet werden kann), für eine Dauer von 2 Stunden einer erhöhten Temperatur ausgesetzt. Nach Abkühlen wurde das Nitrobenzol gaschromatographisch auf den Gehalt an hochsiedenden Verunreinigungen hin analysiert. Die Ergebnisse sind in Tabelle 1 dargestellt und zeigen, dass allein aufgrund der thermischen Belastung hochsiedende Verbindungen entstanden sind, und dass somit Nitrobenzol nicht beliebig hohen Temperaturen ausgesetzt werden kann, wenn es hochsiederfrei erhalten werden soll.

**Tabelle 1**

| | | | |
|---|---|---|---|
| Temperatur der Temperung [°C] | 240 | 260 | 280 |
| Temperdauer [h] | 2 | 2 | 2 |
| Anteil an Hochsiedern [Flächen-ppm] | 193 | 303 | 589 |

### Beispiele 3 bis 5 zur Verwendung von Nitrobenzol unterschiedlichen Salzgehaltes in der Hydrierung zu Anilin (nicht erfindungsgemäß)

Als Versuchsanlage für die Beispielreaktionen dient ein 500 mm langes Reaktionsrohr aus Edelstahl. Durch diesen Reaktor wird ein Kreisgasstrom geleitet, der mittels eines Wärmetauschers auf 250 °C erwärmt wird. Mittels Dosierpumpen wird Nitrobenzol zu einer Düse gefördert und im Kreisgasstrom fein zerstäubt, wo es dann verdampft. Wasserstoff wird in einem Wärmetauscher vorerwärmt und dem Kreisgas vor dieser Düse zudosiert. Die Wasserstoff-Versorgung wird durch einen Massedurchflussregler geregelt. Die Belastung des im Reaktionsrohr befindlichen Katalysators wurde in allen Beispielversuchen auf einen Wert von 1,0 g_{Nitroaromat}/(ml_{Katalysator} · h) eingestellt, und das Wasserstoff : Nitrobenzol-Verhältnis im Reaktor wurde auf ca. 80 : 1 festgelegt.

Auf einem Sieb innerhalb des Reaktionsrohrs wird eine 400 mm hohe Schüttung des Katalysators platziert. Nach Austritt aus dem Reaktor wird das Reaktionsprodukt mit Wasser gekühlt. Die nicht flüchtigen Bestandteile werden so auskondensiert und in einem nachgeschalteten Abscheider von den gasförmigen Komponenten getrennt. Die flüssigen Bestandteile werden aus dem Abscheider in den Produktsammelbehälter geführt und dort aufgefangen (Glasbehälter). Vor dem Sammelbehälter befindet sich eine Probennahmestelle, an der in regelmäßigen Zeitabständen Proben des Produkts gezogen werden können. Diese werden gaschromatographisch analysiert. Die Standzeit des Katalysators entspricht der Zeit vom Beginn der Reaktion bis kein vollständiger Umsatz des Nitrobenzols mehr erreicht wird und an der Probennahmestelle > 0,1 % Nitrobenzol im Produkt mittels Gaschromatographie detektiert werden.

In den Beispielen 3 bis 5 wurde jeweils ein Nitrobenzol eingesetzt, das ca. 250 ppm an organischen Verunreinigungen enthält und damit die erfindungsgemäßen Anforderungen hinsichtlich des Gehaltes an organischen Verunreinigungen (< 500 ppm) erfüllt. Die Nitrobenzolqualitäten in den Beispielen 3 bis 5 unterscheiden sich jedoch hinsichtlich ihres Salzgehaltes (exemplifiziert durch den Gehalt an Natrium-Ionen).

Die Beispiele wurden mit dem Katalysatorsystem 9 g/ l_{Träger} Pd, 9 g/ l_{Träger} V, 3 g/ l_{Träger} Pb auf α-Aluminiumoxid durchgeführt (siehe EP 0 011 090 A1). Es wurden unterschiedlich gealterte und vorbehandelte Katalysatoren dieses Katalysatorsystems eingesetzt; die Reaktionsversuche wurden jeweils abgebrochen, wenn die Standzeit des Katalysators erreicht war.

### Beispiel 3 (nicht erfindungsgemäß): Verwendung von Nitrobenzol mit einem Gehalt an Natrium-Ionen von < 0,1 ppm

Frischer Katalysator wurde im Reaktionsrohr platziert und zunächst mit Stickstoff, dann mit Wasserstoff gespült. Im Anschluss wurde der Katalysator über einen Zeitraum von 48 h bei 240 °C mit 1000 l/h Wasserstoff beaufschlagt. Dann wurde verdampftes Nitrobenzol auf den Katalysator geleitet. Die Nitrobenzolbelastung wurde langsam auf den gewünschten Wert von 1 g_{Nitroaromat}/(ml_{Katalysator} · h) erhöht, sodass die Temperatur im Reaktor nicht über 450 °C anstieg, und die Wasserstoffzugabe wurde so eingestellt, dass das molare Verhältnis von Wasserstoff : Nitrobenzol 80 : 1 betrug. Sobald kein vollständiger Umsatz von Nitrobenzol mehr erfolgte (mehr als 0,1 % Nitrobenzol im Reaktionsprodukt), wurde die Edukteinspeisung beendet und der Reaktor mit Stickstoff inertisiert. Bei 270 °C wurden dann im Luftstrom Verkokungen abgebrannt, bis im Abgas weniger als 0,2 Vol.-% CO₂ detektiert werden konnten. Dieser Zyklus aus Reaktion und Regeneration des Katalysators wurde insgesamt 3 mal (Beispiele 3a bis 3c) durchgeführt. Die Standzeiten betrugen jeweils 983 h, 964 h und 968 h.

### Beispiel 4 (Vergleichsbeispiel): Verwendung von Nitrobenzol mit einem Gehalt an Natrium-Ionen von 0,2 ppm

Frischer Katalysator wurde im Reaktionsrohr platziert und zunächst mit Stickstoff, dann mit Wasserstoff gespült. Im Anschluss wurde der Katalysator über einen Zeitraum von 48 h bei 240 °C mit 1000 l/h Wasserstoff beaufschlagt. Dann wurde verdampftes Nitrobenzol auf den Katalysator geleitet. Die Nitrobenzolbelastung wurde langsam auf den gewünschten Wert von 1,0 g_{Nitroaromat}/(ml_{Katalysator} · h) erhöht, sodass die Temperatur im Reaktor nicht über 450 °C anstieg, und die Wasserstoffzugabe wurde so eingestellt, dass das molare Verhältnis von Wasserstoff: Nitrobenzol 80 : 1 betrug. Sobald kein vollständiger Umsatz von Nitrobenzol mehr erfolgte (mehr als 0,1 % Nitrobenzol im Reaktionsprodukt), wurde die Edukteinspeisung beendet und der Reaktor mit Stickstoff inertisiert. Bei 270 °C wurden dann im Luftstrom Verkokungen abgebrannt, bis im Abgas weniger als 0,2 Vol.-% CO₂ detektiert werden konnten. Dieser Zyklus aus Reaktion und Regeneration des Katalysators wurde insgesamt 3 mal durchgeführt (Beispiele 4a bis 4c). Die Standzeiten betrugen jeweils 953 h, 848 h und 736 h und waren damit - insbesondere im 2. und 3. Zyklus - deutlich schlechter als beim Einsatz eines erfindungsgemäß hergestellten Nitrobenzols.

### Beispiel 5 (Vergleichsbeispiel): Verwendung von Nitrobenzol mit einem Gehalt an Natrium-Ionen von 0,4 ppm

Frischer Katalysator wurde im Reaktionsrohr platziert und zunächst mit Stickstoff, dann mit Wasserstoff gespült. Im Anschluss wurde der Katalysator über einen Zeitraum von 48 h bei 240 °C mit 1000 l/h Wasserstoff beaufschlagt. Dann wurde verdampftes Nitrobenzol auf den Katalysator geleitet. Die Nitrobenzolbelastung wurde langsam auf den gewünschten Wert von 1,0 g_{Nitroaromat}/(ml_{Katalysator} · h) erhöht, sodass die Temperatur im Reaktor nicht über 450 °C anstieg, und die Wasserstoffzugabe wurde so eingestellt, dass das molare Verhältnis von Wasserstoff: Nitrobenzol 80 : 1 betrug. Sobald kein vollständiger Umsatz von Nitrobenzol mehr erfolgte (mehr als 0,1 % Nitrobenzol im Reaktionsprodukt), wurde die Edukteinspeisung beendet und der Reaktor mit Stickstoff inertisiert. Bei 270 °C wurden dann im Luftstrom Verkokungen abgebrannt, bis im Abgas weniger als 0,2 Vol.-% CO₂ detektiert werden konnten. Dieser Zyklus aus Reaktion und Regeneration des Katalysators wurde insgesamt 3 mal durchgeführt (Beispiele 5a bis 5c). Die Standzeiten betrugen jeweils 946 h, 768 h und 605 h und waren damit nochmals schlechter als in Beispiel 4.

Die folgende Tabelle stellt die Resultate der Beispiele 3 bis 4 noch einmal gegenüber:

**Tabelle 2**

| *nicht Erfindungsgemäß* | | *Vergleich* | | | |
|---|---|---|---|---|---|
| Beispiel | Standzeit [h] | Beispiel | Standzeit [h] | Beispiel | Standzeit [h] |
| 3a | 983 | 4a | 953 | 5a | 946 |
| 3b | 964 | 4b | 848 | 5b | 768 |
| 3c | 968 | 4c | 736 | 5c | 605 |

### Beispiel 6 (erfindungsgemäß): Energierückgewinnung bei der Kondensation

Mittels einer Aspen-Simulation wurde die Massen- und Energiebilanz für den zusätzlichen Reinigungsschritt d) für das Nitrobenzol mittels Verdampfung und Kondensation berechnet. Einem kontinuierlich betriebenen Verdampfer werden 50 t/h Nitrobenzol mit einer Temperatur von 183 °C zugeführt. Bei einem absoluten Systemdruck von 500 mbar werden 90 % der Feedmenge (d. h. 90 Massen-% des weiter aufgereinigten Nitrobenzols) unter Nutzung von 20 bar-Dampf verdampft. Die verbleibenden 10 % werden aus dem Verdampfer ausgeschleust und in die saure Wäsche zurückgeführt. Die für die Verdampfung benötige Energie beträgt 4,7 MW bzw. 8,6 t/h 20 bar-Dampf.

Die Brüden werden in einem nachgeschalteten Wärmetauscher kondensiert, der auf der Prozessseite bei 490 mbar absolut betrieben wird. Zur Kühlung wird dabei Kondensat mit 100 °C Vorlauftemperatur eingesetzt, dass zur Erzeugung von 6 bar-Dampf genutzt wird. Im Kondensationsschritt wird eine Energie von 4,7 MW freigesetzt. Da das Kondensat noch von 100 °C auf die Verdampfungstemperatur von 159 °C vorgewärmt werden muss, werden in diesem Apparat 7,2 t/h 6 bar-Dampf erzeugt.

Durch die Nutzung der im Kondensator anfallenden Wärmeenergie kann die für die Verdampfung des Nitrobenzols benötigte Energie komplett wiedergewonnen werden. Der Druck auf der Prozessseite wird dabei so gewählt, dass das MNB mit der höheren der verfügbaren Druckstufen des Dampfes verdampft werden kann und im Kondensator Dampf der niedrigeren der verfügbaren Druckstufen erzeugt werden kann.

## Patentansprüche

1. Verfahren zur Herstellung von Nitrobenzol durch
a) Nitrierung von Benzol mit Salpetersäure oder Gemischen aus Salpetersäure und Schwefelsäure und anschließende Abtrennung überschüssiger in der Nitrierung eingesetzter Säure, wobei Roh-Nitrobenzol erhalten wird,
b) Waschen des Roh-Nitrobenzols aus Schritt a) mittels mindestens jeweils einer sauren, alkalischen und neutralen Wäsche, wobei nach Abtrennung der in der letzten Wäsche eingesetzten Waschflüssigkeit ein vorgereinigtes Nitrobenzol erhalten wird, welches neben Nitrobenzol mindestens noch Leichtsieder enthält,
c) Abtrennung von Leichtsiedern aus dem vorgereinigten Nitrobenzol aus Schritt b) in einem Destillationsapparat durch Verdampfung der Leichtsieder, wobei das Sumpfprodukt Nitrobenzol an Leichtsiedern abgereichert und auf diese Weise weiter aufgereinigt wird,
d) teilweise Abtrennung von Nitrobenzol aus dem weiter aufgereinigten Nitrobenzol aus Schritt c) in einem Destillationsapparat durch teilweise Verdampfung von Nitrobenzol, wobei Rein-Nitrobenzol dem Destillationsapparat gasförmig entnommen und anschließend kondensiert wird, und
e) Rückführung des nicht verdampften Teils des weiter aufgereinigten Nitrobenzols aus Schritt d) in eine beliebige Stelle von Schritt b).

2. Verfahren nach Anspruch 1, bei dem in Schritt d) absolute Drücke im Bereich zwischen 150 mbar und 1000 mbar eingehalten werden und bei dem die in der Kondensation des Rein-Nitrobenzols in Schritt d) abzuführende Wärme zur Gewinnung von Dampf eingesetzt wird.

3. Verfahren nach Anspruch 1 oder 2, bei dem der Destillationsapparat
in Schritt c) eine Rektifizierkolonne ist, welche unter teilweisem Rücklauf von Leichtsiedern betrieben wird, und
in Schritt d) ein Apparat ohne trennwirksame Einbauten ist.

4. Verfahren nach Anspruch 3, bei dem der Destillationsapparat in Schritt d) ohne Rücklauf kondensierten Rein-Nitrobenzols betrieben wird.

5. Verfahren nach Anspruch 1, bei dem in den Schritten c) und d) derselbe Destillationsapparat eingesetzt wird und dieser eine Trennwandkolonne ist.

6. Verfahren nach Anspruch 1, bei dem in der neutralen Wäsche in Schritt b) eine Elektrophorese eingesetzt wird.

## Claims

1. Process for the production of nitrobenzene by
a) nitration of benzene with nitric acid or mixtures of nitric acid and sulfuric acid and subsequent separation of excess acid used in the nitration, wherein crude nitrobenzene is obtained,
b) washing of the crude nitrobenzene from step a) by means of at least one of each of an acid, alkaline and neutral washing, wherein there is obtained after separation of the wash fluid used in the last washing a pre-purified nitrobenzene which, in addition to containing nitrobenzene, also contains at least low boilers,
c) separation of low boilers from the pre-purified nitrobenzene from step b) in a distillation apparatus by evaporation of the low boilers, wherein the bottom product nitrobenzene is depleted of low boilers and thus further purified,
d) partial separation of nitrobenzene from the further purified nitrobenzene from step c) in a distillation apparatus by partial evaporation of nitrobenzene, wherein pure nitrobenzene is removed from the distillation apparatus in gaseous form and is subsequently condensed, and
e) return of the non-evaporated portion of the further purified nitrobenzene from step d) into any desired point of step b).

2. Process according to claim 1, in which absolute pressures in the range from 150 mbar to 1000 mbar are maintained in step d) and in which the heat to be dissipated in the condensation of the pure nitrobenzene in step d) is used to generate steam.

3. Process according to claim 1 or 2, in which the distillation apparatus
in step c) is a rectification column which is operated with partial reflux of low boilers, and
in step d) is an apparatus without separation-active internals.

4. Process according to claim 3, in which the distillation apparatus in step d) is operated without reflux of condensed pure nitrobenzene.

5. Process according to claim 1, in which the same distillation apparatus is used in steps c) and d) and the distillation apparatus is a dividing wall column.

6. Process according to claim 1, in which an electrophoresis is used in the neutral washing in step b).

## Revendications

1. Procédé pour la préparation de nitrobenzène par
a) nitruration de benzène par de l'acide nitrique ou des mélanges d'acide nitrique et d'acide sulfurique et séparation consécutive de l'acide en excès utilisé dans la nitruration, avec obtention de nitrobenzène brut,
b) lavage du nitrobenzène brut de l'étape a) au moyen à chaque fois d'au moins un lavage acide, alcalin et neutre, en obtenant, après la séparation du liquide de lavage utilisé dans le dernier lavage, un nitrobenzène prépurifié, qui contient au moins encore, outre le nitrobenzène, une fraction légère,
c) séparation de la fraction légère du nitrobenzène prépurifié de l'étape b) dans un appareil de distillation par évaporation de la fraction légère, le produit de fond, le nitrobenzène, étant épuisé en fraction légère et ainsi purifié davantage,
d) séparation partielle de nitrobenzène du nitrobenzène purifié davantage de l'étape c) dans un appareil de distillation par évaporation partielle de nitrobenzène, du nitrobenzène pur étant prélevé sous forme gazeuse de l'appareil de distillation et ensuite condensé, et
e) recyclage de la partie non évaporée du nitrobenzène purifié davantage de l'étape d) en un site quelconque de l'étape b).

2. Procédé selon la revendication 1, dans lequel on maintient, dans l'étape d), des pressions absolues dans la plage entre 150 mbars et 1000 mbars et dans lequel la chaleur à évacuer dans la condensation du nitrobenzène pur dans l'étape d) est utilisée pour la production de vapeur.

3. Procédé selon la revendication 1 ou 2, dans lequel l'appareil de distillation dans l'étape c) est une colonne de rectification qui est exploitée avec un reflux partiel de la fraction légère et dans l'étape d), il s'agit d'un appareil sans inserts actifs en séparation.

4. Procédé selon la revendication 3, dans lequel l'appareil de distillation dans l'étape d) est exploité sans reflux du nitrobenzène pur condensé.

5. Procédé selon la revendication 1, dans lequel on utilise, dans les étapes c) et d) le même appareil de distillation et celui-ci est une colonne à paroi de séparation.

6. Procédé selon la revendication 1, dans lequel on utilise une électrophorèse lors du lavage neutre dans l'étape b).
